# EUROPÄISCHE PATENTSCHRIFT

(11) **EP 1 349 544 B1**
(45) Veröffentlichungstag und Bekanntmachung des Hinweises auf die Patenterteilung: **05.04.2006**
(21) Anmeldenummer: 01988039.2
(22) Anmeldetag: 18.12.2001
(51) Int. Cl.: A61K 9/70

(54) **AUFKLEBER MIT VERFLÜSSIGUNGSMITTEL FÜR NATÜRLICHE ATEMWEGSSEKRETE**
ADHESIVE LABEL WITH FLUIDIFYING AGENTS FOR NATURAL AIRWAY SECRETIONS
AUTOCOLLANT COMPORTANT DES AGENTS DE FLUIDIFICATION DE SECRETIONS NATURELLES DES VOIES RESPIRATOIRES

(30) Priorität: 19.12.2000 DE 10063378
(43) Veröffentlichungstag der Anmeldung: 08.10.2003
(73) Patentinhaber: LABTEC GESELLSCHAFT FüR TECHNOLOGISCHE FORSCHUNG UND ENTWICKLUNG MBH, 40764 Langenfeld (DE)
(72) Erfinder: CORDES, Günter, 42799 Leichlingen (DE); VOLLMER, Ulrike, 41542 Dormagen (DE)
(74) Vertreter: Klickow, Hans-Henning
(86) Internationale Anmeldenummer: PCT/EP2001/014945
(87) Internationale Veröffentlichungsnummer: WO 2002/049623

(56) Entgegenhaltungen:
- DE-A- 19 712 359
- DE-C- 19 706 824

## Beschreibung

Erkältungskrankheiten sind eine weitverbreitete Unpässlichkeit. Sie sind gekennzeichnet durch die unangenehmen Folgen der Verstopfung der oberen Atemwege durch eine erheblich gesteigerte Menge an endogenen Sekreten, hervorgerufen durch die Aktivität von Viren und/oder Bakterien. Somit besteht eine sofortige Linderung für den befallenen Körper, wenn der Abfluß der Sekrete erleichtert wird.

Es ist seit Alters her bekannt, dass die Inhaltsstoffe von Pflanzen, die reich an etherischen Ölen sind, zu solch einer Abflusserleichterung geeignet sind. Solche etherischen Öle können als Tee oder in Kapseln (z.B. Gelomyrtol®, Pohl-Boskamp) eingenommen werden, verursachen dabei aber auch oft Reizungen an Magen und Galle. Deshalb wird oft die topische Anwendung in Form von Salben verwendet, die etherische Öle enthalten und die der Patient vorwiegend auf der Brust applizieren soll, damit die Wirkstoffe durch die Haut und über die Atemwege in den Körper gelangen. Entsprechende Präparate sind Wick VapoRub® (Wick Pharma), Bronchoforton® (Plantorgan) und Pinimenthol® (Spitzner). Die Dauer der Wirkstofffreisetzung ist allerdings auf ca. 1 - 2 Stunden begrenzt. Ein weiteres Problem der Salbenapplikation besteht in der Kontamination der Hände mit den schleimhautreizenden etherischen Ölen. Um einen Augenkontakt zu vermeiden, ist es unerlässlich, dass der Patient sich nach Anwendung die Hände wäscht.

Um diesen Nachteil zu umgehen, wurden Trägersysteme aus Vlies oder Gewebe entwickelt, die die Wirkstoffe aufnehmen und eine einfachere Applikation erlauben. Diese werden entweder in die Nähe des Körpers gelegt (DE 4204222, DE 4007275, DE 3911617, DE 3823395) oder auf die Haut aufgeklebt (DE 3540515) und setzen dann die etherischen Öle über einen längeren Zeitraum sowohl zur Hautseite hin als auch in die Atmosphäre frei. In anderen Entwicklungen ist das Wirkstoffreservoir zwischen zwei für den Wirkstoff durchlässigen Folien eingeschlossen (DE 3902981, DE 3216609).

Die Anwendung sowohl von Salben als auch von vorgenannten Systemen bringt die eigentlich für die Inhalation vorgesehenen Wirkstoffe auch in Kontakt mit der Haut. Die Reizung der Haut durch verschiedene etherische Öle (Rosmarinöl, Terpentinöl, Campher) ist bekannt und wird sogar zur Behandlung rheumatischer Erkrankungen ausgenutzt. Bei der Therapie von Erkältungskrankheiten mit etherischen Ölen ist diese Begleitwirkung jedoch unerwünscht, lässt sich aber bei den herkömmlichen Salbenzubereitungen und vorgenannten Reservoirsystemen nicht verhindern.

Die vorliegende Erfindung stellt ein System dar, mit dem die genannten Nachteile vermieden werden können. Es handelt sich dabei um einen Aufkleber, der ein Verflüssigungsmittel enthält, welches mit Hilfe der endogenen Körperwärme in die natürlichen Körperöffnungen der oberen Atemwege eindringt und dort zu einer Verflüssigung der erkältungsbedingten Sekrete führt.

Die der Erfindung zugrundeliegende Aufgabe wird durch einen Aufkleber gelöst, der dadurch gekennzeichnet ist, dass er auf körpernah getragene Kleidung aufklebbar ist und ein Verflüssigungsmittel aufweist, das vom aufgeklebten Aufkleber an die Umgebung des Kleidungsträgers abgegeben wird und in die natürlichen Körperöffnungen der oberen Luftwege eindringen und erkältungsbedingt gestaute Atemwegssekrete verflüssigen kann.

Der erfindungsgemäße Aufkleber kann gekennzeichnet sein durch eine Klebeschicht und/oder eine Schicht für Klettenhaftung, eine entfernbare Schutzschicht für die Klebeschicht oder Klettenhaftungs-Schicht und eine Vliesschicht mit einem Gehalt an Verflüssigungsmittel, wobei das Verflüssigungsmittel aus einer sich selbst verflüssigenden Mischung aus Eucalyptusöl und Campher besteht.

Ferner kann der erfindungsgemäße Aufkleber gekennzeichnet sein durch ein Verflüssigungsmittel, das zu Beginn der Anwendung eine Initialdosis und danach über einen längeren Zeitraum eine Erhaltungsdosis an Verflüssigungsmittel zur Verflüssigung der erkältungsbedingt gestauten Atemwegssekrete abgeben kann, wobei die Abgaberate in mg Verflüssigungsmittel/Vliesschicht-Fläche/Stunde der Initialdosis größer als die der Erhaltungsdosis ist.

Ferner kann der erfindungsgemäße Aufkleber dadurch gekennzeichnet sein, dass das Verflüssigungsmittel über die beiden ersten Stunden nach Beginn der Anwendung eine Initialdosis von 100 bis 300 mg/Stunde, vorzugsweise von etwa 150 mg/Stunde, und danach über mindestens 6 Stunden eine Erhaltungsdosis von 10 bis 30 mg/Stunde, vorzugsweise von etwa 15 mg/Stunde, zur Verflüssigung der erkältungsbedingt gestauten Atemwegssekrete abgeben kann.

Ferner kann der erfindungsgemäße Aufkleber dadurch gekennzeichnet sein, dass die Klebeschicht ausschließlich aus den Monomeren Methylacrylat, 2-Ethylhexylacrylat und Acrylsäure durch radikalische Copolymerisation unter Zusatz eines Vernetzers gewinnbar ist.

Ferner kann der erfindungsgemäße Aufkleber dadurch gekennzeichnet sein, daß die Klebeschicht mit Aluminiumacetylacetonat als Vernetzer gewinnbar ist, insbesondere in einer Menge von 0,04 bis 1 % (bezogen auf das Gewicht aller Monomeren).

Ferner kann der erfindungsgemäße Aufkleber dadurch gekennzeichnet sein, daß die Klebeschicht und die Vliesschicht in nassem, d.h. lösungsmittelhaltigem Zustand miteinander verbunden worden sind und danach getrocknet worden ist.

Ferner kann der erfindungsgemäße Aufkleber gekennzeichnet sein durch synthetische Spinnvliese als Vliesschicht (Trägervlies), insbesondere mit einem Flächengewicht von 70 bis 130 g/m².

Ferner kann der erfindungsgemäße Aufkleber gekennzeichnet sein durch Polyester, Viskose, Trevira, Dralon oder Modal als Material des synthetischen Spinnvlieses.

Ferner kann der erfindungsgemäße Aufkleber gekennzeichnet sein durch eine flauschig anmutende und/oder weiße oder farbige Vliesschicht.

Ferner kann der erfindungsgemäße Aufkleber gekennzeichnet sein durch eine Mischung etherischer Öle als Verflüssigungsmittel, wobei die Mischung ein etherisches Öl, das bei der Temperatur körpernah getragener Kleidung, insbesondere im Bereich von 30 bis 34 °C, nicht-niederviskos oder fest vorliegt, und zusätzlich ein etherisches Öl umfaßt, das bei der Temperatur körpernah getragener Kleidung flüssig vorliegt, wobei die Mischung der Öle bei der Temperatur körpernah getragener Kleidung ebenfalls flüssig vorliegt, ohne dass andere Hilfsstoffe erforderlich sind.

Ferner kann der erfindungsgemäße Aufkleber gekennzeichnet sein durch eine Mischung etherischer Öle auf pflanzlicher Basis, die zur Sekretolyse von Atemwegssekreten zweckdienlich sind, als Verflüssigungsmittel.

Ferner kann der erfindungsgemäße Aufkleber gekennzeichnet sein durch eine Mischung von etherischen Ölen aus pflanzlichen Bestandteilen, deren Inhaltsstoff oder Hauptinhaltsstoff aus der Gruppe der Terpene ausgewählt ist, vorzugsweise aus der Gruppe der Monoterpene, insbesondere aus der durch 1,8-Cineol = Eucalyptol, Campher, Camphen, Menthol, a-Terpineol, Thymol, Pinen und Limonen gebildeten Gruppe.

Ferner kann der erfindungsgemäße Aufkleber gekennzeichnet sein durch Eucalyptusöl als Verflüssigungsmittel oder als einer seiner Bestandteile.

Ferner kann der erfindungsgemäße Aufkleber dadurch gekennzeichnet sein, dass das Verflüssigungsmittel eine Mischung aus Eucalyptusöl und Campher als etherische Öle enthält oder daraus besteht, vorzugsweise im Gewichtsverhältnis Eucalyptusöl:Campher = 3:1.

Ferner kann der erfindungsgemäße Aufkleber gekennzeichnet sein durch eine Größe von 20 bis 200 cm² und vorzugsweise 30 bis 60 cm².

Ferner kann der erfindungsgemäße Aufkleber dadurch gekennzeichnet sein, dass sich ein Aufkleber oder dass sich mehrere Aufkleber, die für eine akute Erkältung ausreichend sind, in einer Verpackung befinden, die bevorzugt gasundurchlässig ist.

Die Penetration von Arzneistoffen in natürliche Öffnungen der Körperoberfläche der Atemwege ist weitgehend durch die physikochemischen Eigenschaften der Substanz bestimmt. Hierbei spielen im wesentlichen der Dampfdruck und die Siedetemperatur bzw. die Flüchtigkeit einer Substanz eine Rolle. Hier wurde nun überraschend gefunden, dass das eutektische und sich selbst verflüssigende Gemisch aus dem flüssigen Eucalyptusöl und dem festen, kristallisiert vorliegenden Campher, in Kombination von etwa 3:1, so ausgezeichnet aus dem Aufkleber heraus in die Körperöffnungen der Atemwege eindringt und außerdem dort zu einer Verflüssigung der Sekrete führt. Es ist nicht mehr erforderlich, zusätzlich zu dem Aufkleber weitere etherische Öle einzusetzen und es ist kein weiteres Vehikel wie Terpentinöl, Alkohol, Vaseline etc. erforderlich wie in all den anderen topischen Salbenformulierungen, um die Inhaltsstoffe an die Atemwege zu transportieren. Es ist bekannt, dass Terpentinöl die verflüssigende Wirkung verstärkt; allerdings wurde auch festgestellt, dass die Migration des Terpentinöls so stark ist, das es selbst durch die Packmittel kriecht, weshalb aus Stabilitäts- und Sicherheitsgründen auf den Zusatz verzichtet wurde.

Allein durch Anbringen des Aufklebers auf die Kleidung des durch Erkältung beeinträchtigten Körpers werden ein Kontakt der ansonsten reizenden etherischen Öle mit sämtlichen Schleimhäuten (Auge, Magen) und anderen Körperoberflächen (Haut) vermieden und trotzdem die sekretverstopften Atemwege erreicht. Diese Sekrete bestehen aus Schleimen, die über Disulfidbrücken an den Proteinpolymeren der Sekrete verfügen. Es hat sich nun gezeigt, dass die alleinige Trennung der Disulfidbrücken z.B. durch Acetylcystein (Fluimucil®-Produkte, Zambon) nicht so zuverlässig zur gewünschten Erleichterung führt wie die Hydratisierung des Mucus, wie sie durch Eucalyptusöl als auch bekanntermaßen durch Guaifenesin erreicht wird. Unterstützend wirkt hier der Campher, der zu einem Kältereiz an den Schleimhäuten führt und damit dem Entzündungszeichen Wärme (Hyperämie) entgegenwirkt.

Es ist wichtig, dass dann, wenn etherische Öle in unverdünnter Form zur Inhalation abgegeben werden, die Abgabe der Öle nicht schlagartig erfolgt, weil dann die etherischen Öle in zu konzentrierter Form freiwerden und zu einem zweiphasischen Umkehreffekt führen können, der unerwünschtsein kann. Die etherischen Öle sollten so stark verdünnt werden, dass der Geruch nur schwach wahrnehmbar ist (Boyd u. Sheppard, 1970). Dies wird durch die Zusammensetzung des Aufklebers (Einbringen der Öle in ein Saugvlies) und die Wahl der Körpertemperatur von 30-34°C optimal erreicht, wie das Abdampfverhalten bei in vitro-Messung verdeutlicht. Es sind keine weiteren Zusätze wie Verdünnung mit Ethanol, Vaselin o.ä. erforderlich. Über die Steuerung durch Vlies und Temperatur kann eine Abgabe in 2 Phasen bzw. Geschwindigkeiten erreicht werden, nämlich eine höhere Rate in den ersten beiden Stunden (Initialdosis mit ca. 150 mg Öl/Stunde) und eine niedrigere, sogenannte Erhaltungsdosis (mit ca. 15 mg Öl/Stunde) nach 2 Stunden, die noch über mind. 6 Stunden anhält. Damit ist der Aufkleber besonders gut für die Anwendung über Nacht auf der Nachtwäsche geeignet.

Wichtig bei der Herstellung solcher Aufkleber mit derart wirksamen Verflüssigungssystemen ist, dass die Verflüssigungsmittel nicht die Polymere des Aufklebers angreifen und verflüssigen. Es wurde nun herausgefunden, dass die Verwendung eines ganz speziellen Acrylat-Copolymer in Verbindung mit einer definierten Menge an Vernetzer zur Antagonisierung eventueller Verflüssigungen des Klebers zu einem stabilen Produkt führt, ohne andere Schutzstoffe wie Sperrfolien, Aluminiumbedampfungen oder ähnliches zusetzen zu müssen, eine optimale Haftung auf verschiedenen Oberflächen der Kleidung (natürliche Fasern wie Wolle oder Baumwolle, als auch synthetische Materialien wie Polyester, Polyamid etc.) ermöglicht und rückstandslos entfernbar ist. Eine besonders gute Verbindung der Klebematrix mit dem Saugvlies wird erreicht, indem im nassen, d.h. lösungsmittelhaltigem Zustand die Verbindung zwischen beiden Schichten hergestellt wird und anschliessend getrocknet wird. Dann kann auch keine Ablösung oder Fädenziehen durch den Zusatz an verflüssigenden etherischen Ölen erfolgen.

Als Material zur Aufnahme der etherischen Öle ist ein saugendes, etwas dickeres Vlies geeignet. Am besten geeignet sind Vliese ("Nonwovens") von einem Flächengewicht von mind. 70 bis 130 g/m², die z.B. aus Polyesterarten oder Viskose hergestellt werden. Als Schutzfolie für die klebende Seite des Aufklebers kann eine dem Fachmann bekannte silikonisierte Polyesterfolie, z.B. Hostaphan RN 100 von Diafoil, Hoechst, Germany, easy/easy, eingesetzt werden, die nicht zu dünn sein sollte (mind. 36 µm Schichtdicke, vorzugsweise 100 µm Schichtdicke), damit der Aufkleber von 30 bis 200 cm², vorzugsweise von 30 bis 60 cm² noch gut in der Praxis anwendbar ist.

Die nachfolgenden Ausführungsbeispiele dienen zur näheren Erläuterung der Erfindung:

### Beispiel 1:

Um 100 m² Rollenware Aufkleber herzustellen, gibt man zu 28,858 kg einer 35 %igen (m/m) Lösung eines Acrylat-Klebstoffes (Durotak 87-2852, National Starch & Chemical B.V., NL-Zutphen) 0,051 kg Aluminiumacetylacetonat. Durch halbstündiges Rühren wird die Lösung homogenisiert und anschließend mit einem Rakel auf einer silikonisierten, 100 µm starken Polyesterfolie (FL 2000 100 µ 1-S, Rexam Release B.V., NL-Apeldoorn) in einer Naßschichtdicke von 400 µm ausgestrichen. Vor der Trocknung wird mit Paramoll N260/100 (Polyestervlies der Fa. Lohmann, D-Andernach) bedeckt und anschließend getrocknet (15 min bei 70°C). Das so entstandene homogene Laminat wird durch Schneiden auf einzelne Aufkleber der Größe 60 cm² gebracht. Es werden unmittelbar vor der Verpackung in Verbundpackstoff-Siegelbeutel mittels Spritzdüsen 500 mg der etherischen Ölmischung aus Eucalyptusöl:Campher 3:1 aufgebracht.

Man erhält einen Aufkleber, der einen Kleberanteil von 102 g/m² und 20% Eucalyptusöl sowie 6,7% Campher enthält.

### Beispiel 2:

Um 100 m² Rollenware Aufkleber herzustellen, gibt man zu 28,858 kg einer 35 %igen (m/m) Lösung eines Acrylat-Klebstoffes (Durotak 87-2852, National Starch & Chemical B.V., NL-Zutphen) 0,051 kg Aluminiumacetylacetonat. Durch halbbstündiges Rühren wird die Lösung homogenisiert und anschließend mit einem Rakel auf einer silikonisierten, 100 µm starken Polyesterfolie (FL 2000 100 µ 1-S, Rexam Release B.V., NL-Apeldoorn) in einer Naßschichtdicke von 400 µm ausgestrichen. Vor der Trocknung wird mit TWE-Vlies 100 (Viskosevlies der Fa. TWE, D-Emsstätten) bedeckt und anschließend getrocknet (15 min bei 70°C). Das so entstandene homogene Laminat wird durch Stanzen auf einzelne Aufkleber der Größe 59 cm² gebracht. Es werden unmittelbar vor der Verpackung in Verbundpackstoff-Siegelbeutel mittels Spritzdüsen 500 mg der etherischen Ölmischung aus Eucalyptusöl:Campher 3:1 aufgebracht. Man erhält einen Aufkleber, der einen Kleberanteil von 102 g/m² und 20% Eucalyptusöl sowie 6,7% Campher enthält.

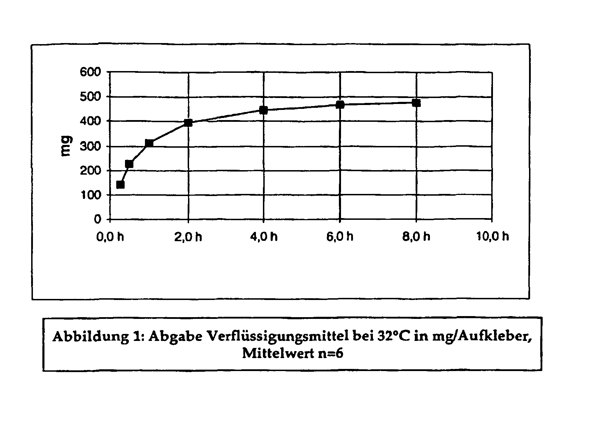

## Patentansprüche

1. Aufkleber, der auf körpernah getragene Kleidung aufklebbar ist und ein Verflüssigungsmittel aufweist, das vom aufgeklebten Aufkleber an die Umgebung des Kleidungsträgers abgegeben wird und in die natürlichen Körperöffnungen der oberen Luftwege eindringen und erkältungsbedingt gestaute Atemwegssekrete verflüssigen kann, wobei das Verflüssigungsmittel aus einer sich selbst verflüssigenden Mischung aus Eucalyptusöl und Campher besteht.

2. Aufkleber nach Anspruch 1, **gekennzeichnet durch** eine Klebeschicht und/oder eine Schicht für Klettenhaftung, eine entfernbare Schutzschicht für die Klebeschicht oder Klettenhaftungs-Schicht und eine Vliesschicht mit einem Gehalt an Verflüssigungsmittel, insbesondere einer Mischung aus zwei oder mehr etherischen Ölen als Verflüssigungsmittel.

3. Aufleber nach Anspruch 1 oder 2, **gekennzeichnet durch** ein Verflüssigungsmittel, das zu Beginn der Anwendung eine Initialdosis und danach über einen längeren Zeitraum eine Erhaltungsdosis an Verflüssigungsmittel zur Verflüssigung der erkältungsbedingt gestauten Atemwegssekrete abgeben kann, wobei die Abgaberate in mg Verflüssigungsmittel/Vliesschicht-Fläche/Stunde der Initialdosis größer als die der Erhaltungsdosis ist.

4. Aufkleber nach Anspruch 3, **dadurch gekennzeichnet, dass** das Verflüssigungsmittel über die beiden ersten Stunden nach Beginn der Anwendung eine Initialdosis von 100 bis 300 mg/Stunde, vorzugsweise von etwa 150 mg/Stunde, und danach über mindestens 6 Stunden eine Erhaltungsdosis von 10 bis 30 mg/Stunde, vorzugsweise von etwa 15 mg/Stunde, zur Verflüssigung der erkältungsbedingt gestauten Atemwegssekrete abgeben kann.

5. Aufkleber nach einem der Ansprüche 2 bis 4, **dadurch gekennzeichnet, dass** die Klebeschicht ausschließlich aus den Monomeren Methylacrylat, 2-Ethylhexylacrylat und Acrylsäure durch radikalische Copolymerisation unter Zusatz eines Vernetzers gewinnbar ist..

6. Aufkleber nach Anspruch 5, **dadurch gekennzeichnet, daß** die Klebeschicht mit Aluminiumacetylacetonat als Vernetzer gewinnbar ist, insbesondere in einer Menge von 0,04 bis 1 % (bezogen auf das Gewicht aller Monomeren).

7. Aufkleber nach Anspruch 5, **dadurch gekennzeichnet, daß** die Klebeschicht und die Vliesschicht in nassem Zustand miteinander verbunden worden sind und danach getrocknet worden ist.

8. Aufkleber nach einem der vorhergehenden Ansprüche, **gekennzeichnet durch** synthetische Spinnvliese als Vliesschicht (Trägervlies), insbesondere mit einem Flächengewicht von 70 bis 130 g/m².

9. Aufkleber nach Anspruch 8, **gekennzeichnet durch** Polyester, Viskose, Trevira, Dralon oder Modal als Material des synthetischen Spinnvlieses.

10. Aufkleber nach einem der Ansprüche 8 oder 9, **gekennzeichnet durch** eine flauschig anmutende und/oder weiße oder farbige Vliesschicht.

11. Aufkleber nach einem der Ansprüche 2 bis 14, **dadurch gekennzeichnet, dass** das Verflüssigungsmittel aus einer Mischung aus Eucalyptusöl und Campher als etherische Öle im Gewichtsverhältnis Eucalyptusöl:Campher = etwa 3:1 besteht.

12. Aufkleber nach einem der vorhergehenden Ansprüche, **gekennzeichnet durch** eine Größe von 20 bis 200 cm² und vorzugsweise 30 bis 60 cm².

13. Aufkleber nach einem der vorhergehenden Ansprüche, **dadurch gekennzeichnet, dass** sich ein Aufkleber oder dass sich mehrere Aufkleber, die für eine akute Erkältung ausreichend sind, in einer Verpackung befinden, die bevorzugt gasundurchlässig ist.

## Claims

1. Sticker which can be affixed to clothing worn next to the body and has a liquefying agent which is released from the affixed sticker to the vicinity of the wearer of the clothing and can penetrate the natural body orifices of the upper respiratory tract and liquefy the respiratory tract secretions accumulated as a result of a cold, the liquefying agent consisting of a self-liquefying mixture of eucalyptus oil and camphor.

2. Sticker according to Claim 1, **characterised by** an adhesive layer and/or a layer for burr adhesion, a removable protective layer for the adhesive layer or burr adhesion layer and a nonwoven layer with a content of liquefying agent, in particular a mixture of two or more essential oils as liquefying agent.

3. Sticker according to Claim 1 or 2, **characterised by** a liquefying agent which on commencement of the application can release an initial dose and thereafter over a longer period of time a maintenance dose of liquefying agent to liquefy the respiratory tract secretions accumulated as a result of a cold, the release rate in mg liquefying agent/nonwoven surface area/hour of the initial dose being greater than that of the maintenance dose.

4. Sticker according to Claim 3, **characterised in that** the liquefying agent can release in the first two hours after commencement of application an initial dose of 100 to 300 mg/hour, preferably approximately 150 mg/hour, and thereafter over at least 6 hours a maintenance dose of 10 to 30 mg/hour, preferably approximately 15 mg/hour, to liquefy the respiratory tract secretions accumulated as a result of a cold.

5. Sticker according to one of Claims 2 to 4, **characterised in that** the adhesive layer is obtainable exclusively from the monomer methyl acrylate, 2-ethylhexyl acrylate and acrylic acid by radical copolymerisation with addition of a cross-linking agent.

6. Sticker according to Claim 5, **characterised in that** the adhesive layer is obtainable with aluminium acetyl acetonate as cross-linking agent, in particular in a quantity of 0.04 to 1% (based on the weight of all monomers).

7. Sticker according to Claim 5, **characterised in that** the adhesive layer and the nonwoven layer have been bonded together in the wet state and subsequently dried.

8. Sticker according to one of the preceding claims, **characterised by** synthetic spin-bonded nonwovens as nonwoven layer (support nonwoven), in particular with a weight per unit area of 70 to 130 g/m².

9. Sticker according to Claim 8, **characterised by** polyester, viscose, Trevira, Dralon or modal as material of the synthetic spin-bonded nonwoven.

10. Sticker according to one of Claims 8 or 9, **characterised by** a fleecy appearance and/or white or coloured nonwoven layer.

11. Sticker according to one of Claims 2 to 14, **characterised in that** the liquefying agent consists of a mixture of eucalyptus oil and camphor as essential oils in a ratio by weight of eucalyptus oil : camphor = approximately 3 : 1.

12. Sticker according to one of the preceding claims, **characterised by** a size of 20 to 200 cm² and preferably 30 to 60 cm².

13. Sticker according to one of the preceding claims, **characterised in that** there is one sticker or that there are several stickers which are sufficient for an acute cold in a pack which is preferably impermeable to gas.

## Revendications

1. Etiquette adhésive, qui peut être collée sur un vêtement porté, côté corps et qui présente un agent de liquéfaction, qui se dégage de l'étiquette adhésive collée, dans l'environnement du porteur du vêtement et peut pénétrer dans les ouvertures corporelles naturelles des voies respiratoires supérieures et peut liquéfier les sécrétions des voies respiratoires engorgées par le froid, où l'agent de liquéfaction consiste en un mélange auto-liquéfiant d'huile d'eucalyptus et de camphre.

2. Etiquette adhésive suivant la revendication 1, **caractérisée par** une couche de colle et/ou une couche agrippante, une couche protectrice éliminable pour la couche de colle ou la couche agrippante, et une couche de voile avec une teneur en agent de liquéfaction, en particulier un mélange de deux ou plusieurs huiles éthérées comme agent de liquéfaction.

3. Etiquette adhésive suivant la revendication 1 ou 2, **caractérisée par** un agent de liquéfaction, qui peut dégager au début de l'utilisation, une dose initiale et ensuite, sur une période plus longue, une dose d'entretien en agent de liquéfaction pour liquéfier les sécrétions des voies respiratoires engorgées par le froid, où la vitesse de dégagement en mg d'agent de liquéfaction/surface de la couche de voile/heure de la dose initiale est supérieure à celle de la dose d'entretien.

4. Etiquette adhésive suivant la revendication 3, **caractérisée en ce que** l'agent de liquéfaction peut se dégager lors des deux premières heures après le commencement de l'utilisation, une dose initiale de 100 à 300 mg/heure, de préférence d'environ 150 mg/heure, et ensuite, lors d'au moins 6 heures, une dose d'entretien de 10 à 30 mg/heure, de préférence d'environ 15 mg/heure, pour liquéfier les sécrétions des voies respiratoires engorgées par le froid.

5. Etiquette adhésive suivant l'une des revendications 2 à 4, **caractérisée en ce que** la couche de colle peut être obtenue exclusivement, à partir des monomères acrylate de méthyle, acrylate de 2-éthylhexyle et acide acrylique, par copolymérisation radicalaire en utilisant un agent de réticulation.

6. Etiquette adhésive suivant la revendication 5, **caractérisée en ce que** le couche de colle peut être obtenue avec l'acétylacétonate d'aluminium comme agent de réticulation, en particulier en une quantité de 0,04 à 1% (sur base du poids de tous les monomères).

7. Etiquette adhésive suivant la revendication 5, **caractérisée en ce que** le couche de colle et la couche de voile sont reliées l'une à l'autre à l'état humide et ensuite, sont séchées.

8. Etiquette adhésive suivant l'une des revendications précédentes, **caractérisée par** un voile filable synthétique comme couche de voile (voile support), en particulier avec un poids surfacique de 70 à 130 g/m².

9. Etiquette adhésive suivant la revendication 8, **caractérisée par** du polyester, de la viscose, du Trevira, du Dralon ou du Modal comme matériau du voile filable synthétique.

10. Etiquette adhésive suivant l'une des revendications 8 ou 9, **caractérisée par** une couche de voile à effet moelleux et/ou blanc ou coloré.

11. Etiquette adhésive suivant l'une des revendications 2 à 14, **caractérisée en ce que** l'agent de liquéfaction consiste en un mélange d'huile d'eucalyptus et de camphre comme huiles éthérées, en un rapport pondéral huile d'eucalyptus:camphre d'environ 3:1.

12. Etiquette adhésive suivant l'une des revendications précédentes, **caractérisée par** une taille de 20 à 200 cm² et de préférence, de 30 à 60 cm².

13. Etiquette adhésive suivant l'une des revendications précédentes, **caractérisée en ce qu'**une étiquette adhésive ou plusieurs étiquettes adhésives, qui sont suffisantes pour un refroidissement important, se trouvent dans un emballage, qui est de préférence, imperméable aux gaz.
